# EUROPEAN PATENT APPLICATION

(11) **EP 1 714 963 A1**
(43) Date of publication of application: **25.10.2006**
(21) Application number: 06112734.6
(22) Date of filing: 18.04.2006
(51) Int. Cl.: C07D 257/04, A61K 31/40, A61P 9/12

(54) **Process for the Preparation of Valsartan and its Intermediates**

(30) Priority: 19.04.2005 IN MU04902005
(71) Applicant: IPCA Laboratories Limited, Kandivli (West), Numbai 400 067 (Maharashtra) (IN)
(72) Inventor: Kumar, Ashok, (West), 400 053, Mumbai, Maharashtra (IN); Nimbalkar, Manmohan Madhavrao, 400025, Mumbai, Maharashtra (IN); Barve, Sanjay Govind, 400 004, Mumbai, Maharashtra (IN); Metil, Dattatray Shamrao, Evershine City 401 208 Thane Maharashtra (IN); Shimpukade, Bharat Dinkar, 411 033, Pune, Maharashtra (IN); Kushwaha, Lavkesh, 307, Shri Trimurti Apt., (East), 401 105, Thane, Maharashtra (IN); Kelkar, Rahul Suresh, Shivaji Road, 400 068 Mumbai Maharashtra (IN)
(74) Representative: Brand, Thomas Louis

(57) **Abstract**

The present invention concerns a process for preparing valsartan of Formula I: 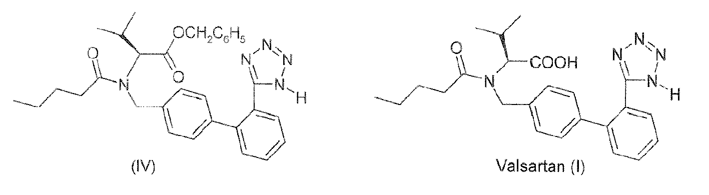 comprising purifying intermediate benzyl valsartan of Formula IV by crystallizing said benzyl valsartan of lower purity from a first solvent which is a ternary mixture comprising a hydrophilic solvent, a non-polar protic solvent and water; recovering benzyl valsartan from said ternary mixture followed by crystallizing benzyl valsartan from a second solvent comprising a non-polar aprotic solvent or polar aprotic solvent or their mixture; and recovering benzyl valsartan substantially free of organotin impurity; and converting said benzyl valsartan of into valsartan

## Description

### Field of the Invention:

The present invention relates to an improved process for the preparation of a known pharmaceutical agent, valsartan, and its intermediates in substantially pure enantiomeric form.

### Background of the Invention:

(S)-N-(1-Carboxy-2-methyl-prop-1-yl)-N-pentanoyl-N-[2'-(1H-tetrazol-5-yl)-biphenyl-4-ylmethyl]-amine commonly known as valsartan has the following structure (Formula I):

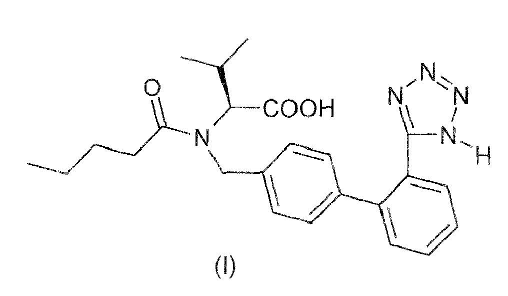

Valsartan is a member of the class of agents termed angiotensin-II (AT) receptor antagonists having effective anti-hypertensive activity with an excellent profile of safety and tolerability. Activation of AT receptors in the outer membrane of vascular smooth muscle cells of the heart and arteries causes the tissues to constrict. AT-I receptors are activated by an octa-peptide, angiotensin-II. Angiotensin-II helps to maintain constant blood pressure despite fluctuations in a person's state of hydration, sodium intake and other physiological variables. Angiotensin-II also performs the regulatory tasks of inhibiting excretion of sodium by the kidneys, inhibiting nor-ephedrin reuptake and stimulating aldosterone biosynthesis. By inhibiting angiotensin-II binding to AT receptors, valsartan disrupts the vasoconstriction mediated by AT receptors.

Valsartan is therefore a non-peptide angiotensin-II antagonist. Valsartan inhibits the actions of angiotensin-II on its receptors, thus preventing the increase of blood pressure produced by the hormone-receptor interactions. Hence it is used in the treatment of cardiovascular complaints such as hypertension and heart failure. Comparative trial studies have shown that valsartan is as effective as angiotensin-converting enzymes, (ACE) inhibitors, calcium-channel blockers and â-blockers, and is generally better tolerated. Valsartan is marketed as the free acid under the trade name DIOVAN. However, its combination with diuretics such as hydrochlorothiazide has specific advantages as an anti-hypertensive agent.

The synthesis of valsartan and its intermediates of formula II, III and IV are reported in patent (US patent no. 5,399,578) and Bioorganic & Medicinal Chemistry Letters, vol. 4, pp 29-34, 1994, via the following method as shown in Scheme 1:

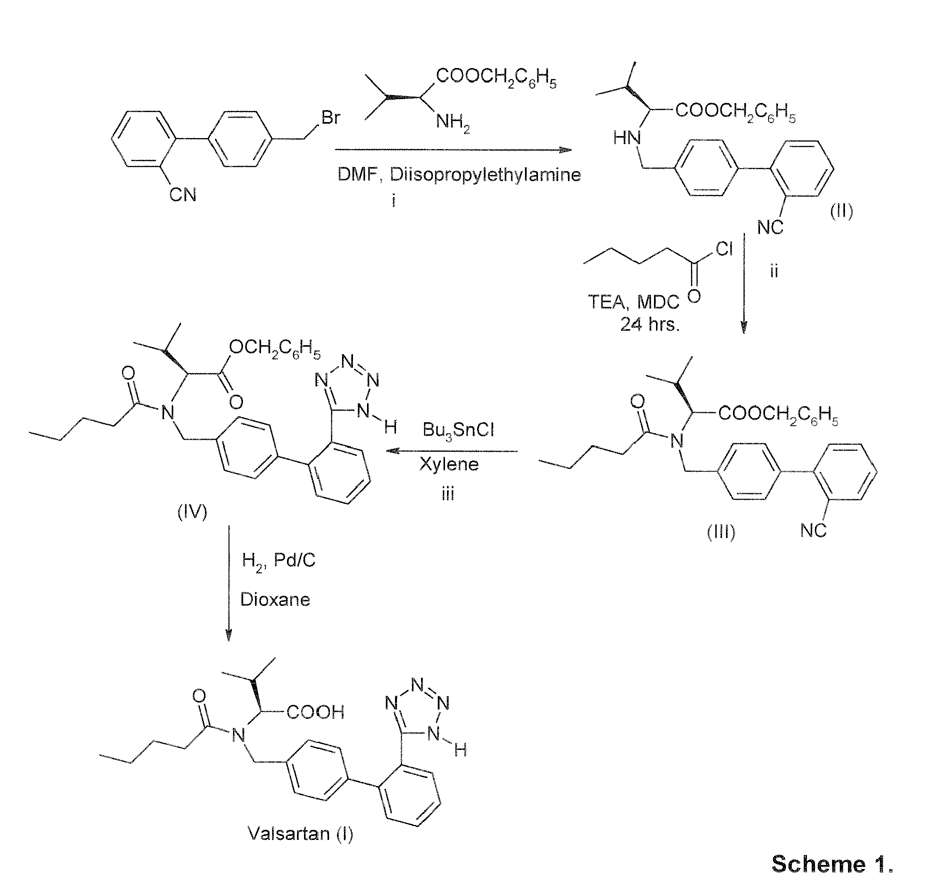

This process appears in some respects to be practical but involves many disadvantages from the point of view of purity/yield of valsartan and its intermediates. The major problems are incomplete reactions, longer reaction times, contamination of valsartan with a number of impurities or starting material/intermediates and lower chiral purity of valsartan obtained.

Particular disadvantages of the synthesis disclosed in US Patent No. 5,399,578 include long reaction time and incomplete reaction of (S)-N-[(2'-cyanobiphenyl-4-yl)methyl]-(L)-valine benzyl ester or salts thereof with valeroyl chloride in chlorinated solvents in the preparation of (S)-N-[(2'-cyanobiphenyl-4-yl)methyl]-N-valeroyl-(L)-valine benzyl ester of formula III. This can result in contamination of compound II. This impurity in turn is carried forward in subsequent reaction steps and results in valsartan of low purity.

A further disadvantage of this prior art method is contamination by large amounts of various impurities, especially organotin, in the penultimate intermediate of valsartan namely, (S)-N-[(2'-(1H-tetrazol-5-yl)biphenyl-4-yl)methyl]-N-valeroyl-(L)-valine benzyl ester of the formula IV (benzyl valsartan), during the tetrazole formation of Formula III. An effective purification or means to remove the organotin by-product and other impurities from benzyl valsartan are not described or disclosed in the '578 patent. As a result the catalyst (palladium -charcoal) gets poisoned in the presence of the organotin impurity during the debenzylation of benzyl valsartan (IV) to valsartan, and necessitates a very high loading of palladium- charcoal for completing the reaction. This is coupled with susceptibility of valsartan or its intermediate to partial racemization during the reaction conditions or purification processes as described in the '578 patent. This makes the prior art process impractical for a high purity valsartan for clinical use. Racemization herein means the process of a relatively pure enantiomer of a substance becoming a mixture of enantiomeric forms. Valsartan is used in the enantiomerically pure (S)-N-[(2'-(1H-tetrazol-5-yl)biphenyl-4-yl)methyl]-N-valeroyl-(L)-valine form in pharmaceutical compositions. This prevents a synthetic chemist from using drastic conditions to push the reactions for completion when reactions are slow, and poses considerable challenges to design milder reaction conditions

Conventional processes generally employ solvents like DMF during the preparation of the compounds of formula II and formula III and there is a current need to avoid or minimize the use of such environmentally unfriendly materials.

There is also a need in the art for an improved and cost-effective synthetic process for the preparation of valsartan and precursors thereof in its enantiomerically pure form.

### Objects of the invention

It is an objective of the present invention to overcome or ameliorate at least one of the disadvantages of the prior art or to provide a useful alternative.

It is an object of the present invention in its preferred form to provide an industrial process for the preparation of valsartan substantially in its pure enantiomeric form.

Other objectives of this invention include the establishment of suitable purification methods to remove the organotin by-product from benzyl valsartan, to overcome the incomplete reactions during preparation of the intermediate compounds, to speed up the reactions and to provide suitable environmentally friendly solvents in a process for the manufacture of valsartan.

### Summary of the invention:

Accordingly, the present invention relates to an improved method for the preparation of valsartan having an enantiomeric purity of at least 99.8% or more.

In one process of the present invention, the intermediate compound of Formula II is prepared by reacting a compound of Formula (IIa) with compound of Formula (IIb) in a heterogeneous solvent system/medium comprising a mixture of water and an organic solvent in presence of an inorganic base, and isolating the product (II) as hydrochloride salt or free base. The said reaction may be optionally performed in presence of a catalyst.

In another process of the present invention, the compound II is acylated with valeroyl chloride in the presence of an organic base in a solvents selected from non-polar solvents like toluene, xylene etc., the reaction proceeding substantially to completion (e.g. by conversion of at least 99.8% of the starting compound II) in a short period of time.

In yet another process of the present invention the benzyl valsartan intermediate substantially free of the organotin impurity is provided by first reacting Compound III with tributyl tin azide in toluene or xylene, followed by purifying the isolated crude benzyl valsartan contaminated with organotin impurity from a first solvent that is a ternary solvent mixture, characterized by combination of a hydrophilic organic solvent selected from C₁ to C₄ alcohol, a non-polar organic solvent like hexane or toluene or the like and water, followed by crystallizing from a second solvent such as a non-polar organic solvent or a polar aprotic solvent or mixtures thereof.

In a further aspect, the present invention provides a process for debenzylation of benzyl valsartan substantially free of organotin impurity using palladium carbon characterized by a significantly lower catalyst loading at ambient temperature in a hydrophilic organic solvent selected from C₁ to C₄ alcohol followed by crystallizing valsartan of enantiomeric purity of at least 99.8 % from a solvent mixture such as ethyl acetate and hexane or ethyl acetate and ether at a temperature below 60°C.

### Detailed description of the invention:

The invention will now be described in detail in connection with certain preferred and optional embodiments, so that various aspects thereof may be more fully understood and appreciated.

The present invention provides a process for preparation of valsartan and its intermediates in substantially pure enantiomeric form.

In a first process step, the compound of the general Formula IIa is reacted with compound of general formula IIb in the presence of an inorganic base and optionally in presence of a catalyst. In a preferred process of the present invention the above reaction step is carried out in a heterogeneous solvent mixture comprising water and a non-polar hydrocarbon solvent.

In the general formula IIa:

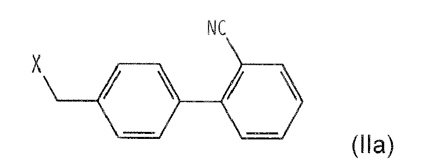

X is any halogen atom. Preferably the halogen is bromine.

In the general formula IIb or a salt thereof:

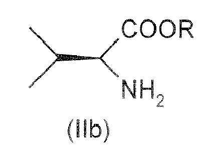

R represents any carboxyl protecting group. However, the preferred carboxyl protecting group is methyl or benzyl group. In the reaction the compound IIb may be used as its acid salt, preferably a hydrochloric acid salt or a p-toluene sulphonic acid salt and more preferably as p-toluene sulphonic acid salt. If an acid salt is employed in the reaction an excess amount of base is used, which is required to neutralize the salt to free compound Ilb.

The heterogeneous solvent mixture comprises water and an organic solvent preferably a hydrocarbon solvent such as toluene, xylene. The organic solvent and water mixture comprises, based on the total volume of the solvent mixture, from about 40% to 80% of non-polar organic solvent and about 60% to 20% of water. Preferably the reaction is carried out in a mixture of non-polar hydrocarbon solvent and water comprising, based on the total volume of the solvent mixture, from about 40% to 60% of non-polar hydrocarbon solvent and about 60% to 40% of water.

As described above the preferred non-polar hydrocarbon solvents are selected from C₆ - Cg aromatic hydrocarbons or C₅ - C₈ aliphatic hydrocarbons or C₅ - C₈ alicyclic hydrocarbons, or suitable mixtures of two or more thereof. Other hydrocarbons useful in the practice of the present invention will be apparent to the skilled artisan. The preferred non-polar solvent is selected from, but is not limited to, toluene, xylene, hexane, cyclohexane or mixtures of two or more thereof. The most preferred non-polar solvent is toluene.

The basic material used in the reaction is an inorganic base such as a carbonate or bicarbonate salt of an alkali metal and preferably the alkali metal salt is sodium carbonate, potassium carbonate or sodium bicarbonate, or mixtures of two or more thereof.

The reaction may optionally be carried out in the presence of a catalyst. Preferred catalysts are selected from potassium iodide, sodium iodide, or a phase-transfer catalyst such as tetrabutyl ammonium bromide, tetrabutyl ammonium chloride or a combination of phase-transfer catalyst and sodium/potassium iodide.

The reaction is carried out at a temperature from an ambient temp to the reflux temperature of the reaction mixture. More preferably heat is used to effect the completion of reaction at a temperature of about 40°C to 60°C and the reaction completes in a period of about 20 hours to 30 hours.

On completion of the reaction, the intermediate compound II is isolated from the reaction mixture by phase separation & water washing, and acidification of the organic layer. The reaction product (S)-N-[(2'-cyanobiphenyl-4-yl)methyl]-(L)-valine benzyl ester of formula II, gets separated out in the form of its hydrochloride salt after acidification of the reaction mixture using hydrogen chloride, (gaseous hydrochloric acid or aqueous hydrochloric acid) to a pH of 1-4.

The precipitated (S)-N-[(2'-cyanobiphenyl-4-yl)methyl]-(L)-valine benzyl ester hydrochloride shows a purity above 95% with an yield of about 85%. The (S)-N-[(2'-cyanobiphenyl-4-yl)methyl]-(L)-valine benzyl ester hydrochloride is used as such in subsequent reaction or may be isolated as its free base by neutralization procedure known in the art.

In a second step of the present invention, the (S)-N-[(2'-cyanobiphenyl-4-yl)methyl]-(L)-valine benzyl ester (Formula II) or its hydrochloride salt is reacted with a compound of formula Ilc as shown below in presence of a base like diisopropylethylamine.

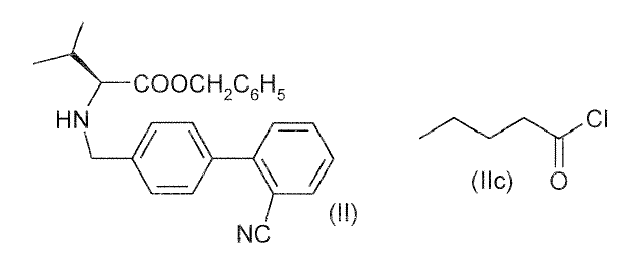

According to the present invention the above process step, reaction of (S)-N-[(2'-cyano- biphenyl-4-yl)methyl]-(L)-valine benzyl ester or its hydrochloride salt with compound IIc, is carried out in a non-polar hydrocarbon solvent. The preferred non-polar solvent is selected from C₆ - C₉ aromatic hydrocarbon or C₅ - C₈ aliphatic hydrocarbon or C₅ - C₈ alicyclic hydrocarbon and the solvent is preferably toluene.

The reaction is carried out at a temperature between 0°C to the reflux temperature and preferably between 20°C to the reflux temperature of the solvent. Most preferably the reaction is carried out between 25°C - 35°C and the reaction completes in a span of 1 to 5 hours.

In the third step, the cyano group in compound of formula III is converted into tetrazole ring system by reaction with tributyltin azide to produce the penultimate intermediate of valsartan called benzyl valsartan. Preferably the tributyltin azide is added in three lots. Toluene is used as solvent and the reaction is effected at reflux temperature. In a preferred embodiment of the present invention the stage III product on water washings is directly employed in the tetrazole formation when the solvent is toluene or xylene. After the completion of tetrazole formation, the benzyl valsartan is released from the tributyl tin complex by passing dry hydrochloric acid gas to the reaction mixture, toluene is decanted off and the residue is taken in water immiscible solvent, washed with water till free of acid. The compound of formula IV is isolated as oil in crude form.

Preferably the second step and third step is carried out *in situ* without isolating the product of second step.

The present invention provides a purification method for effective removal of the organotin impurity from benzyl vafsartan of the formula (IV)

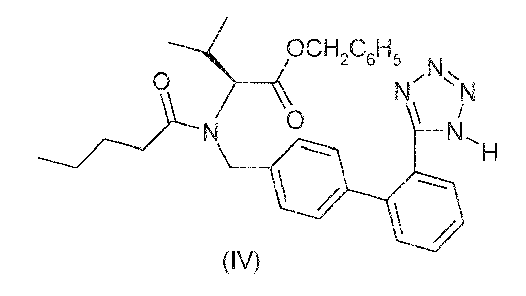

The purification process of the present invention includes a first crystallization of benzyl valsartan from a ternary solvent mixture comprises a hydrophilic organic solvent, a non-polar organic solvent and water, and a second crystallization from a polar aprotic solvent or non-polar organic solvent or mixtures thereof, preferably second crystallization solvent is a binary solvent mixture comprises of polar aprotic solvent and non-polar organic solvent.

The hydrophilic organic solvent used in the ternary solvent mixture in the first purification step is selected from C₁ to C₄ alcohols especially isopropanol. The non-polar organic solvents are selected from hydrocarbon solvents like hexane, toluene, cyclohexane or the like. The preferred composition of the ternary mixture is as follows: the ratio of hydrophilic organic solvent to inert non-polar organic solvent is in the range of about 1:0.5 to 1:100 parts by weight and preferably is in the range of about 1:0.5 to 1:25 parts by weight and more preferably is in the range of about 1:0.5 to 1:10 parts by weight, on the basis of 1 part by weight of compound of Formula IV. The ratio of water to combination of hydrophilic organic solvent and inert non-polar organic solvent is in the range of about 0.5: 1 to 10:1 parts by weight and preferably the ratio is in the range of about 0.5:1 to 5:1 parts by weight, on the basis of 1 part by weight of compound of formula IV.

In a preferred form, water is added to a pre-formed solution of compound of formula IV (Benzyl valsartan) in a mixture of hydrophilic organic solvent and non-polar organic solvent between 0°C to reflux temperature. Preferably the addition is carried out between 25°C - 35°C and the benzyl valsartan precipitates out of the solution. The precipitated compound of formula IV is isolated by filtration or other conventional means at ambient temperature or by further cooling to 0°C.

According to the present invention, benzyl valsartan is further purified from a second solvent, preferably the non-polar organic solvent or polar aprotic solvent or mixtures thereof, to obtain benzyl valsartan substantially free of organotin impurity in high enantiomeric purity.

The non-polar organic solvent is selected from C₆ - C₉ aromatic hydrocarbon, C₅ - C₈ aliphatic hydrocarbon or C₅ - C₈ alicyclic hydrocarbon. The non-polar organic solvents are selected from hydrocarbon solvents like hexane, toluene or the like and the inert polar aprotic solvent is ethyl acetate.

In the process of purification the non-polar organic solvent is added to a solution of benzyl valsartan in polar aprotic solvent between 0°C to reflux temperature.

Preferably the addition of non-polar organic solvent is carried out between 25°C - 35°C to precipitate benzyl valsartan substantially free of organotin impurity. Benzyl valsartan prepared according to this embodiment has a low level of tin content as determined by Atomic Emission Spectroscopy. It contains less than about 1000ppm (parts per million), or preferably less than about 100 ppm or more preferably is substantially free of any detectable tin content as analyzed by atomic emission spectroscopy.

The combination of non-polar organic solvent to ethyl acetate preferably has a ratio in which ethyl acetate to the non-polar organic solvent is in the range of about 1:0 to 1:100 parts by weight and preferably is in the range of about 1:0 to 1:50 parts by weight and more preferably is in the range of about 1:0 to 1:25 parts by weight, on the basis of 1 part by weight of compound of formula IV. The precipitated benzyl valsartan can be separated from the mixture by conventional means such as filtration and can be optionally dried at a temperature below 60°C.

The benzyl valsartan obtained above is subjected to hydrogenation in presence of palladium-charcoal to obtain valsartan. The present invention is characterized by a lower loading of costly palladium-charcoal catalyst in the debenzylation of benzyl valsartan substantially free of organotin impurity. In the presence of organotin impurity, the catalyst gets poisoned and looses the activity and thus necessitates a heavy loading of costly palladium-charcoal catalyst in the prior art.

The present invention has reduced the palladium-charcoal quantity by about 70% (16 times reduction on a wt/wt basis) with respect to the prior art processes. After the completion of the reaction, the product was filtered, concentrated and solution of valsartan in an aqueous basic solution is prepared. The aqueous basic solution may be that of an alkali metal or alkaline earth metal salt such as sodium carbonate, sodium bicarbonate, potassium carbonate, sodium hydroxide or potassium hydroxide. The aqueous layer is washed with chlorinated solvent such as methylene dichloride or ethylene dichloride. The aqueous solution is then rendered acidic using aqueous acidic solutions of acids such as hydrochloric acid, sulfuric acid or acetic acid. Precipitated valsartan is then extracted using an organic solvent such as ethyl acetate or isobutyl methyl ketone or methyl propyl ketone. The organic phase is concentrated under reduced pressure to dryness; residue is suspended / slurry washed with hydrocarbon solvent such as toluene, hexane, cyclohexane, a combination of hydrocarbon solvent with ethyl acetate or diisopropyl ether or a mixture thereof and valsartan can be separated from the solvent mixture by decanting, filtering, centrifuging or using other similar processing methods of isolation of solids from liquids known to a person skilled in the art, or any combination of such separation methods and is optionally followed by a wash with water.

Valsartan after acidification and extraction in above said solvent can also be isolated by slurrification, at a temperature below 60°C, from a premix solvent mixtures such as ethyl acetate-hexane or ethyl acetate-diisopropylether optionally in presence of water. Valsartan can be separated from the mixture by decanting, filtering, centrifuging or using other similar processing methods of isolation of solids from liquids known to a person skilled in the art, or any combination of such separation methods and is optionally followed by a wash with water.

Isolated valsartan is dried, at a temperature below 60°C, by air drying, vacuum drying or fluidized bed drying or using other similar drying methods of solids known to a person skilled in the art, or any combination of such drying methods.

The temperature of reaction as well as isolation and drying is crucial for getting higher enantiomeric excess as at higher temperature valsartan tends to racemize. The valsartan obtained by following the process of the present invention has a purity of at least 99.7% and the R-isomer content is less than 0.10%.

This drastic yield and purity improvement caused by the above-described variations thus lead to an efficient and commercially acceptable synthetic process for the preparation of valsartan.

This higher enantiomeric purity and yield starting from compound of Formula II constitutes a considerable technical advance with respect to the process described in US 5,399,578 patent.

The invention is explained in more detail in the following working examples. The examples, which illustrate improvement in the method according to the invention, have a purely illustrative character and do not limit the extent of the invention in any respect.

### Example 1 : (S)-N-[(2'-cyanobiphenyl-4-yl)methyl]-(L)-valine benzyl ester hydrochloride (Formula II)

4-bromomethyl-2'-cyanobiphenyl (15 g, 0.055 mol), L-valine benzyl ester tosylate (20.9 g, 0.055 mol), potassium carbonate (21 g, 0.152 mol), and potassium iodide (0.21 g) were heated to 50-55°C in toluene (63 mL) and water (63 mL) for 25 hours. After cooling, the two phases separated. The organic layer was water washed (2 × 50 mL) and acidified with hydrochloric acid to pH 1-2; upon which, (S)-N-[(2'-cyanobiphenyl-4-yl)methyl]-(L)-valine benzyl ester hydrochloride crystallized. Precipitated solids were filtered, washed with toluene and dried to give 17.9 gms (75% yield) of (S)-N-[(2'-cyanobiphenyl-4-yl)methyl]-(L)-valine benzyl ester hydrochloride having a purity of 97% as measured by HPLC area percent.

### Example 2 : (S)-N-[(2'-cyanobiphenyl-4-yl)methyl]-(L)-valine benzyl ester hydrochloride (Formula II)

4-bromomethyl-2'-cyanobiphenyl (15 g, 0.055 mol), L-valine benzyl ester tosylate (29.9 g, 0.055 mol), potassium carbonate (21 g, 0.152 mol), and tetrabutyl ammonium bromide (0.21 g) were heated to 50-55°C in xylene (63 mL) and water (63 mL) for 25 hours. After cooling, the two phases separated. The organic layer was water washed and acidified with hydrochloric acid to pH 1-2; upon which, (S)-N-[(2'-cyanobiphenyl-4-yl)methyl]-(L)-valine benzyl ester hydrochloride precipitated. The obtained solids were filtered, washed with toluene and dried to give 17.4 gms (73% yield) of (S)-N-[(2'-cyanobiphenyl-4-yl)methyl]-(L)-valine benzyl ester hydrochloride having a purity of 97% as measured by HPLC area percent.

### Example 3 : (S)-N-[(2'-cyanobiphenyl-4-yl)methyl]-(L)-valine benzyl ester hydrochloride (Formula II)

4-bromomethyl-2'-cyanobiphenyl (4.2 kg, 0.0154 mol), L-valine benzyl ester tosylate (5.85 kg, 0.0154 mol), potassium carbonate (5.88 kg, 0.0426 mol), tetrabutyl ammonium bromide (0.059 kg) and potassium iodide (0.059 kg) were heated to 50-55°C in toluene (17.6 L) and water (17.6 L) for 25 hours. After cooling, the two phases separated. The organic layer was water washed (2 × 14 L) and acidified with hydrochloric acid to pH 1-2; upon which, the (S)-N-[(2'-cyanobiphenyl-4-yl)methyl]-(L)-valine benzyl ester hydrochloride precipitated which was filtered, washed with toluene and dried to give 6.05 kgs (90% yield) of (S)-N-[(2'-cyanobiphenyl-4-yl)methyl]-(L)-valine benzyl ester hydrochloride having a purity of 97% as measured by HPLC area percent.

### Example 4: (S)-N-[(2'-cyanobiphenyl-4-yl)methyl]-N-valeroyl-(L)-valine benzyl ester (Formula III)

To sodium bicarbonate (65 g, 0.774 mol) in water (800 mL) and toluene (400 mL) was added (S)-N-[(2'-cyanobiphenyl-4-yl)methyl]-(L)-valine benzyl ester hydrochloride (210 g, 0.483 mol) at 25°C. The mixture was stirred for an hour; after which, the layers separated. The toluene layer was then washed with a brine solution and dried over sodium sulfate. To the dried toluene layer containing (S)-N-[(2'-cyano biphenyl-4-yl)methyl]-(L)-valine benzyl ester, N,N-diisopropylethylamine (101.8 g, 0.786 mol) was added at 25°C and cooled to 20°C. Valeroyl chloride (80 g, 0.664 mol) was then added over a period of about 30 minutes. After the completion of the reaction by TLC [thin layer chromatography] check, 50 mL water was added and further stirred for 30 minutes. The layers were separated; and 1N hydrochloric acid solution was added to the organic layer at 0°C until the pH reaches about 1-3. The acidified mixture was stirred for 30 minutes; and the layers separated. 10% sodium bicarbonate solution was added to the organic layer until the pH of reaction mass reaches about 7-8. The layers then separated; and the organic layer was washed with 200 mL water, and then with 200 mL brine solution. The organic layer was dried, solvent distilled out under reduced pressure to obtain 228 g (98% yield) of (S)-N-[(2'-cyanobiphenyl-4-yl)methyl]-N-valeroyl-(L)-valine benzyl ester as a brownish oil having a purity of 96% as measured by HPLC area percent.

### Example 5: (S)-N-(1-benzyloxycarbonyl-2-methyl-prop-1-yl)-N-pentanoyl-N-[2'-(1H-tetrazol-5-yl)-biphenyl-4-ylmethyl]-amine (Formula IV)

To a mixture of sodium bicarbonate (65 g, 0.774 mol), water (800 mL) and toluene (400 mL) was added (S)-N-[(2'-cyanobiphenyl-4-yl)methyl]-(L)-valine benzyl ester hydrochloride (210 g, 0.483 mol) at 25°C and stirred for an hour. The layers were separated; and the toluene layer was dried over sodium sulfate. N,N-diisopropylethylamine (101.8 gm, 0.786 mol) was added to the dried toluene layer, containing (S)-N-[(2'-cyano biphenyl-4-yl)methyl]-(L)-valine benzyl ester, at 25°C. It was then cooled down to 15°C; and valeryl chloride (80 g, 0.664 mol) was added over a period of about 60 minutes. Reactrion progress monitored by TLC, on completion of reaction 100 mL water was added and further stirred for 30 minutes. The organic layer separaeted out and washed first with 1N hydrochloric acid solution until the pH was about 1-3, and then washed with 10% aqueous sodium bicarbonate solution until the pH of the washings reaches 7-8 respectively. The toluene layer was further washed with 200 mL water and then with 200 mL brine solution. The organic layer was subsequently dried using sodium sulfate. The dried organic layer was further used as such for tetrazole formation.

To the above dried toluene layer was added sodium azide (70 g, 1.08 mol) and tributyl tin chloride (350 g, 1.07 mol), and heated to reflux. After about 20 hrs, a second lot of sodium azide (10 g, 0.154 mol) and tributyl tin chloride (50 g, 0.154 mol) was added. The reflux continued for 20-24 hrs. After the completion of reaction by TLC check, the reaction mass was cooled to about 15°C. Dry hydrochloric acid gas was then passed through the toluene layer until the decolorization of toluene was observed. The toluene layer was decanted; and the residue was washed with 100 mL of toluene. The residue was then partitioned between methylene chloride and a 10% sodium bicarbonate solution, and stirred at 25-30°C for 30 minutes. The layers then separated; and the organic layer was washed with a brine solution and dried over sodium sulfate. The solvent was distilled off under reduced pressure to obtain 240 g of (S)-N-(1-benzyloxycarbonyl-2-methyl-prop-1-yl)-N-pentanoyl-N-[2'-(1H-tetrazol-5-yl)-biphenyl-4-ylmethyl]-amine [benzyl valsartan] as a brownish oil having HPLC assay purity 91%.

### Example 6:

### Purification of (S)-N-(1-benzyloxycarbonyl-2-methyl-prop-1-yl)-N-pentanoyl-N-[2'-(1H-tetrazol-5-yl)-biphenyl-4-ylmethyl]-amine (Benzyl valsartan)

23 g of the crude (S)-N-(1-benzyloxycarbonyl-2-methyl-prop-1-yl)-N-pentanoyl-N-[2'-(1H-tetrazol-5-yl)-biphenyl-4-ylmethyl]-amine oil obtained in example 5 was taken in 40 mL isopropyl alcohol [IPA] and heated to obtain a clear solution. To the reaction mass was added 200 mL hexane. It was further cooled to about 10-20°C; and 250 ml water was added in about 60 minutes. The mixture was stirred at 25-30°C for 60 minutes. It is filtered, washed with water and dried to give 18 gms (78% yield) of (S)-N-(1-benzyloxycarbony)-2-methyl-prop-1-yl)-N-pentanoyl-N-[2'-(1H-tetrazol-5-yl)-biphenyl-4-ylmethyl]-amine having a purity of 94% as measure by HPLC assay. Melting point (MP)106-108°C;

To 5 g of the above solid was added to 15 mL toluene and heated to obtain a clear solution. 10 mL hexane was then added and cooled to ambient temperature; upon which (S)-N-(1-benzyloxycarbonyl-2-methyl-prop-1-yl)-N-pentanoyl-N-[2'-(1H-tetrazol-5-yl)-biphenyl-4-ylmethyl]-amine crystallized. It was filtered, washed with hexane and dried to give 4.5 gms (90%) of (S)-N-(1-benzyloxycarbonyl-2-methyl-prop-1-yl)-N-pentanoyl-N-[2'-(1H-tetrazol-5-yl)-biphenyl-4-ylmethyl]-amine having purity of 100.46% as measured by HPLC assay with not detectable tin content. MP 115.9-116°C.

### Example 8: Purification of Benzyl valsartan (Formula IV)

1.53 kgs of the crude (S)-N-(1-benzyloxycarbonyl-2-methyl-prop-1-yl)-N-pentanoyl-N-[2'-(1H-tetrazol-5-yl)-biphenyl-4-ylmethyl]-amine (Formula IV) oil prepared according to the example 5 was taken in 2.67 L of isopropyl alcohol and heated to obtain a clear solution. To the reaction mass was added 13.3 L hexane. It was further cooled to about 10-20°C; and 16.67 L water was added in about 60 minutes. The mixture was stirred at 25-30°C for 60 minutes. Precipitate was filtered, washed with water and dried to give 1.2 kgs (78% yield) of (S)-N-(1-benzyloxycarbonyl-2-methyl-prop-1-yl)-N-pentanoyl-N-[2'-(1H-tetrazol-5-yl)-biphenyl-4-ylmethyl]-amine having purity93-96%(HPLC assay). The product has melting point 106-108°C.

1 kg of the above obtained solid was added to 2 L ethyl acetate and heated to obtain a clear solution. 4 L hexane was then added and cooled to 25-30°C; upon which, (S)-N-(1-benzyloxycarbonyl-2-methyl-prop-1-yl)-N-pentanoyl-N-[2'-(1H-tetrazol-5-yl)-biphenyl-4-ylmethyl]-amine crystallized out. The precipitate was filtered, washed with hexane and dried to give 3.5 kgs (70% yield) of (S)-N-(1-benzyloxycarbonyl-2-methyl-prop-1-yl)-N-pentanoyl-N-[2'-(1H-tetrazol-5-yl)-biphenyl-4-ylmethyl]-amine having purity 100.15% (HPLC assay) with no detectable tin content (analyzed by atomic emission spectroscopy). The product melts at 115.2-115.8°C.

### Example 9 : (S)-N-(1-Carboxy-2-methyl-prop-1-yl)-N-pentanoyl-N-[2'-(1H-tetrazol-5-yl)-biphenyl-4-ylmethyl]-amine (Valsartan)

A solution of 50 g (S)-N-(1-benzyloxycarbonyl-2-methyl-prop-1-yl)-N-pentanoyl-N-[2'-(1H-tetrazol-5-yl)bi-phenyl-4-ylmethyl]-amine in 500 mL methanol was hydrogenated at room temperature with 2.5 g (5% loading) of 5% Pd-C until the completion of reaction. After reaction catalyst was filtered out and crude valsartan was obtained by evaporation of the solvent. It was then partitioned between 500 mL of 10% sodium bicarbonate solution and 200 mL MDC [dichloromethane]. The aqueous phase was separated and rendered acidic. Valsartan was isolated by extraction with ethyl acetate,and crystallization from a mixture of ethyl acetate and hexane. The precipitated valsartan was filtered, washed with hexane and dried to give 34 gms (82% yield) of Valsartan. Purity of valsartan obtained was 99.8% and R-isomer 0.07% as measured by HPLC area percent.

### Example 10:

### (S)-N-(1-Carboxy-2-methyl-prop-1-yl)-N-pentanoyl-N-[2'-(1H-tetrazol-5-yl)-biphenyl-4-ylmethyl]-amine (Valsartan)

A solution of 50 g (S)-N-(1-benzyloxycarbonyl-2-methyl-prop-1-yl)-N-pentanoyl-N-[2'-(1H-tetrazol-5-yl)bi-phenyl-4-ylmethyl]-amine in 300 mL methanol was hydrogenated at room temperature with 1,25 g (2.5 % loading) of 5% Pd-C and further proceeded analogously to example 9. Valsartan was crystallized from a mixture of ethyl acetate and di-isopropyl ether, filtered, washed with di-isopropyl ether and dried to give 29 gms of Valsartan (Yield-70%). Purity of valsartan was 99.83% and R-isomer 0.03% as measured by HPLC area percent.

### Example 11 : Valsartan

A solution of 50 g (S)-N-(1-benzyloxycarbonyl-2-methyl-prop-1-yl)-N-pentanoyl-N-[2'-(1H-tetrazol-5-yl)bi-phenyl-4-ylmethyl]-amine in 500 mL methanol was hydrogenated at room temperature with 1.25 g (2.5% loading) of 5% Pd-C until the completion of reaction. The crude valsartan was obtained by filtration and evaporation of the solvent. The residue was partitioned between 500 mL of 10% sodium bicarbonate solution and 200 mL MDC. The aqueous phase was separated and rendered acidic. Valsartan was isolated by extraction with ethyl acetate. The organic phase was distilled under reduced pressure to dryness. The residue was then suspended in a mixture of ethyl acetate and hexane at ambient temperature, filtered, washed with water, and dried to give 34 gms of Valsartan (yield of 82%). Purity of Valsartan obtained was 99.8% and R-isomer 0.05% by HPLC analysis.

### Example 12 : Valsartan

A solution of 50 g (S)-N-(1-benzyloxycarbonyl-2-methyl-prop-1-yl)-N-pentanoyl-N-[2'-(1H-tetrazol-5-yl)bi-phenyl-4-ylmethyl]-amine in 500 mL methanol was hydrogenated at room temperature with 2.5 gm (5% loading) of 5% Pd-C until the completion of reaction. The crude valsartan was obtained by filtration and evaporation of the solvent, and was partitioned between 500 mL of 10% sodium bicarbonate solution and 200 mL MDC. The aqueous phase was separated and rendered acidic. Valsartan was isolated by extraction with ethyl acetate. The organic phase was distilled under reduced pressure to dryness, slurried in a mixture of ethyl acetate:hexane:water (ratio 2:5:1), filtered, and dried to obain 34 gm (82% yield) of valsartan. Purity of valsartan obatinaed was 99.8% and R-isomer 0.02% as measured by HPLC analysis.

### COMPARATIVE EXAMPLES AS PER US '578 PATENT

### Comparative Example 1

### (S)-N-[(2'-cyanobiphenyl-4-yl)methyl]-N-valeroyl-(L)-valine benzyl ester (Formula III)

6.2 g (15.5 mmol) of (S)-N-[(2'-cyanobiphenyl-4-yl)methyl]-(L)-valine benzyl ester and 8.0 mL N,N-diisopropylethylamine, dissolved in 50 ml of methylene chloride, are treated with 2.3 mL of valeryl chloride with stirring. The reaction mixture was stirred at room temperature for 20-25 hours until the starting amine can no longer be detected by TLC. After evaporating in a water jet vacuum, the reaction mixture was partitioned between 82 mL water and 826 mL ethyl acetate. The organic phase was washed successively with 40 mL each of 2N hydrochloric acid, saturated NaHCO₃ solution, and brine; dried over magnesium sulfate; and evaporated in vacuum. The title compound obtained as brown oil with a purity of 72% as measured by HPLC area percent.

### Comparative Example 2

### (S)-N-(1-benzyloxycarbonyl-2-methyl-prop-1-yl)-N-pentanoyl-N-[2'-(1H-tetrazol-5-yl)-biphenyl-4-ylmethyl]-amine [Benzyl valsartan]

6.6 g (13.6 mmol) of (S)-N-[(2'-cyanobiphenyl-4-yl)methyl]-N-valeroyl-(L)-valine benzyl ester and 6.0 g (18 mmol) of tributyltin azide, in 75 mL of o-xylene, were heated to boiling with stirring for 48 hours. After 24 hours, an additional 2.0 g of tributyltin azide was added. After cooling, the solution was diluted with about 125 mL of toluene, treated with 110 mL 1N potassium hydroxide solution, and stirred for 20 minutes. The aqueous phase was separated and rendered acidic with 1N hydrochloric acid solution. The product was extracted in ethyl acetate. The title compound after evaporation of solvent was obtained as oil with a purity of 86-88% as analyzed by HPLC assay.

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. A process for preparing valsartan of Formula I comprises: 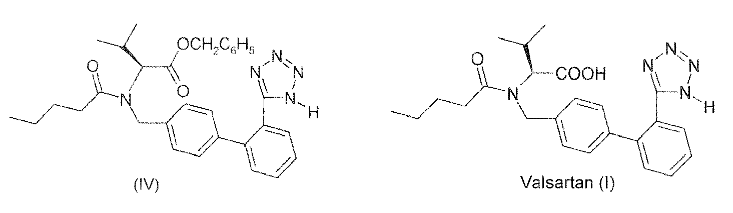
a) purifying intermediate benzyl valsartan of Formula IV by crystallizing said benzyl valsartan from a first solvent comprising a ternary mixture of a hydrophilic solvent, a non-polar protic solvent and water; recovering benzyl valsartan from said ternary mixture followed by re-crystallizing benzyl vaisartan from a second solvent comprising a non-polar aprotic solvent or polar aprotic solvent or mixture thereof; and recovering benzyl valsartan at a higher purity than the intermediate benzyl valsartan which is substantially free of organotin impurity; and
b) converting said benzyl valsartan of step (a) into valsartan.

2. The process as claimed in claim 1, wherein the ternary mixture comprises hydrophilic solvents selected from C₁-C₄ alcohols and non-polar solvents selected from C₆ - C₉ aromatic hydrocarbons, C₅ - C₈ aliphatic hydrocarbons or C₅ - C₈ alicyclic hydrocarbons, or mixtures of two or more thereof.

3. The process as claimed in claim 1 or 2, wherein said ternary solvent mixture has a hydrophilic organic solvent to non-polar organic solvent ratio in the range of 1 : 0.5 to 1 : 30 parts, and wherein the hydrophilic organic solvent and non-polar organic solvent to water ratio is in the range of about 1 : 0.5 to 1 : 10 parts by weight relative to benzyl valsartan.

4. The process as claimed in any one of claims 1 to 3, wherein the hydrophilic solvent comprises isopropanol.

5. The process as claimed in any one of claims 1 to 4, wherein the non-polar solvent comprises hexane or toluene.

6. The process as claimed in any one of claims 1 to 5, wherein the second purification solvent system has a polar aprotic solvent to non-polar organic solvent ratio in the range of about 1 : 0 to 1 : 50 parts by weight relative to benzyl valsartan.

7. The process as claimed in any on of claims 1 to 6, wherein the second crystallizing solvent is selected from ethyl acetate, hexane, toluene, or mixtures of two or more thereof.

8. The process as claimed as claimed in any one of claims 1 to 7, wherein the second crystallization solvent comprises a binary mixture comprising a non-polar aprotic solvent and a polar aprotic solvent.

9. The process as claimed in claim 8, wherein the binary mixture is a combination of ethyl acetate and hydrocarbon solvent.

10. The process as claimed in any one of claims 1 to 9, wherein step (b) comprises
c) hydrogenation of benzyl valsartan obtained from step (b) in presence of palladium carbon **characterized by** catalyst quantity less than 10 % wt/wt relative to benzyl valsartan; and
d) recovering vafsartan.

11. The process as claimed in claim 10, wherein the catalyst quantity is 2.5 - 5 wt % relative to benzyl valsartan.

12. The process as claimed in claim 10 or claim 11, wherein the recovered valsartan is dried below 60 °C.

13. The process as claimed in any one of claims 1 to 12, wherein the benzyl valsartan having less than about 1000 ppm (parts per million) of organotin impurity as measured by atomic emission spectroscopy and less than 0.1% of benzyl (R)-N-(1-Carboxy-2-methyl-prop-1-yl)-N-pentanoyl-N-[2'-(1H-tetrazol-5-yl)-biphenyl-4-ylmethyl]-amine isomer as measured by HPLC area percent.

14. The process as claimed in any one of claims 1 to 13 wherein the valsartan has an enantiomeric purity greater than 99.5%.

15. The process as claimed in any one of claims 1 to 14, wherein the valsartan obtained comprises less than about 0.1% (R)-N-(1-Carboxy-2-methyl-prop-1-yl)-N-pentanoyl-N-[2'-(1H-tetrazol-5-yl)-biphenyl-4-ylmethyl]-amine isomer.

16. The process as claimed in any one of claims 1 to 15, wherein the benzyl valsartan of Formula IV is prepared from a compound of Formula III: 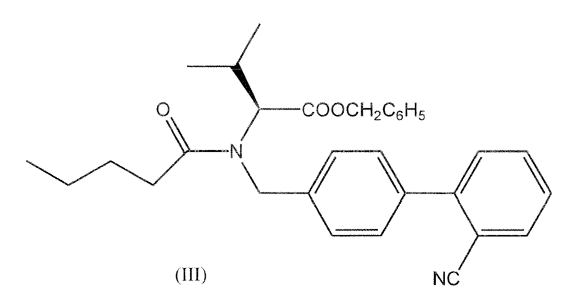 using a tributyltinazide complex in presence of a hydrocarbon solvent.

17. The process as claimed in claim 16, wherein the tributyl tin azide is prepared *in situ* from tributyltinchloride and sodium azide.

18. The process as claimed in any one of claims 1 to 17 wherein the ternary solvent mixture comprises isopropyl alcohol, hexane and water.

19. The process as claimed in any one of claims 1 to 18 wherein the second crystallization solvent is a binary mixture comprising non-polar aprotic solvent and polar aprotic solvent.

20. The process as claimed in claim 19, wherein the second crystallization solvent is a binary mixture comprising ethyl acetate and hexane.

21. A process according to any one of claims 1 to 20 for preparing valsartan of Formula I comprising:
a) reacting 4-bromomethyl-2'-cyanobiphenyl with L-valine benzyl ester or its acid salt **characterized in that** the reaction is carried out in a heterogeneous solvent mixture comprising water and non-polar hydrocarbon solvent, in presence of an inorganic base to obtain a compound of Formula II; 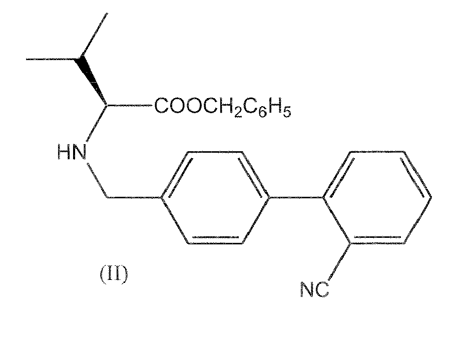
b) reacting the step (a) product with valeroylchloride in presence of diisopropylethylamine **characterized in that** reaction is conducted in solvent selected from toluene or hexane.
c) providing benzyl valsartan of Formula IV from the product of step (b) (Formula III) using an tributyltinazide;
d) purifying said benzyl valsartan **characterized by** crystallizing from a first solvent which is a ternary mixture comprising a hydrophilic solvent, a non-polar solvent and water; recovering benzyl valsartan from said ternary mixture followed by crystallizing benzyl valsartan from a second solvent which is a binary mixture comprising ethyl acetate and hexane; and recovering benzyl valsartan substantially free of tributyltin impurity; and
e) converting said benzyl valsartan of step (d) into valsartan using palladium carbon
f) recovering valsartan having less than 0.1% (R)-N-(1-Carboxy-2-methyl-prop-1-yl)-N-pentanoyl-N-[2'-(1H-tetrazol-5-yl)-biphenyl-4-ylmethyl]-amine isomer.

22. The process as claimed in claim 21, wherein the step (a) is carried out in presence of a catalyst selected from sodium iodide, potassium iodide and a phase-transfer catalyst or a combination thereof.

23. valsartan or benzyl valsartan prepared according to the process of any one of claims 1 to 22 having less than 1000 ppm (parts per million) of organotin impurity as measured by atomic emission spectroscopy and less than 0.1 % of benzyl (R)-N-(1-Carboxy-2-methyl-prop-1-yl)-N-pentanoyl-N-[2'-(1H-tetrazol-5-yl)-biphenyl-4-ylmethyl]-amine isomer as measured by HPLC area percent.

24. valsartan or benzyl valsartan prepared by the process of any one of claims 1 to 23, and having less than about 1000 ppm (parts per million) of organotin impurity as measured by atomic emission spectroscopy.

25. A pharmaceutical composition **characterized in that** a high purity valsartan obtained according to any one of claims 1 to 23 is put into a pharmaceutically acceptable form.

26. A pharmaceutical composition comprising valsartan prepared according to claim 25 for the use in ameliorating (combating) cardiovascular or hypertensive disorders in mammals.

27. A pharmaceutical composition according to claim 25 or 26 further comprising a diuretic agent such as hydrochlorothiazide or chlorthalidone.
